# EUROPÄISCHE PATENTANMELDUNG

(11) **EP 1 747 754 A1**
(43) Veröffentlichungstag der Anmeldung: **31.01.2007**
(21) Anmeldenummer: 06014397.1
(22) Anmeldetag: 11.07.2006
(51) Int. Cl.: A61B 5/055

(54) **Vorrichtung zur NMR-Untersuchung intrakorporaler Körperbereiche sowie deren Verwendung**

(30) Priorität: 26.07.2005 DE 102005034838
(71) Anmelder: Fraunhofer-Gesellschaft zur Förderung der angewandten Forschung e.V., 80686 München (DE)
(72) Erfinder: Volke, Frank, Dipl.-Phys., Dr., 66386 St. Ingbert (DE); König, Karsten, Prof. Dr., 66119 Saarbrücken (DE)
(74) Vertreter: Rösler, Uwe

(57) **Zusammenfassung**

Beschrieben wird eine Vorrichtung zur NMR-Untersuchung intrakorporaler Körperbereiche einer Person mit einer Spulenanordnung, die in unmittelbare Nähe zu einem intrakorporalen Körperbereich bringbar ist, der von einem stationären, magnetischen Polarisationsfeld B₀ sowie von einem RF-Wechselfeld durchsetzt wird.

Die Erfindung zeichnet sich dadurch aus, dass die Spulenanordnung in einer gekapselten, autarken Einheit integriert ist, die eine von der Person verschluckbare Form und Größe aufweist, dass in der gekapselten, autarken Einheit eine Sendeeinheit integriert ist, die mit der Spulenanordnung verbunden ist und von Seiten der Spulenanordnung herrührende Induktionssignale an eine extrakorporal vorgesehene Empfängereinheit drahtlos übermittelt.

## Beschreibung

### Technisches Gebiet

Die Erfindung bezieht sich auf eine Vorrichtung zur NMR-Untersuchung (NMR, Nuclear Magnetic Resonance) intrakorporaler Körperbereiche einer Person mit einer Spulenanordnung, die in unmittelbare Nähe zu einem intrakorporalen Körperbereich bringbar ist, der von einem stationären, magnetischen Polarisationsfeld B₀ sowie von einem RF-Wechselfeld (RF, radio frequency) durchsetzt wird, sowie auf deren Verwendung.

### Stand der Technik

Kernresonanz-spektroskopische Untersuchungen ermöglichen im Anwendungsbereich der Medizin ortsaufgelöste Bilddarstellungen intrakorporaler Körperbereiche, die durch eine Zusammenschau einer Vielzahl von zweidimensionalen Schichtbildern ein dreidimensionales Abbild ganzer Körperbereiche bzw. Organe ermöglichen. Zur Durchführung derartiger NMR-Untersuchungen werden Kernspin-Tomographen eingesetzt, die in der Lage sind, jeweils mit Hilfe groß dimensionierter Magnetspulensysteme starke stationäre magnetische Polarisationsfelder B₀ zu erzeugen, die von lokal wirkenden hochfrequenten Wechselfeldern überlagert werden. Aufgrund innerhalb des zu untersuchenden Körpers auftretenden Wechselwirkungen zwischen den Kernspins von Protonen und dem hochfrequenten Wechselfeld treten im Resonanzfall Absorptionen des Wechselfeldes durch die Protonenspins auf, deren resultierende Echosignale, die so genannten MR-Signale, mit Hilfe einer Empfänger- bzw. MR-Spule, die ebenfalls im Kernspin-Tomographen untergebracht ist, detektiert werden können. Auf der Grundlage von MRI-Verfahren (magnetic resonance imaging) gelingt es über magnetische Gradienten und entsprechende elektromagnetische Anregungsimpulse die absorptionsbedingten MR-Signale, die einer Frequenzkodierung unterliegen und in eine Ortskodierung transformiert werden, als Bildinformation aufzubereiten und auf einem Monitor visuell sichtbar zu machen. Um möglichst hochqualitative MR-Signale zu erhalten, bedarf es am zu untersuchenden Bereich der Ausbildung eines möglichst homogenen HF-Wechselfeldes, das maßgeblich für eine kontrastreiche Bildwiedergabe des untersuchten Körperbereiches verantwortlich ist.

Zu diesem Zwecke ist in der DE 197 46 735 vorgeschlagen worden, eine HF-Spule aus einem flexiblen elektrischen Leiter auf einen intrakorporal einführbaren Katheter, z.B. einem Gefäßkatheter, auf der Oberfläche eines dillatierbaren Ballons zu montieren. Mit Hilfe eines derartig präparierten Katheters lassen sich Wände von Gefäßen im Rahmen von MRT-Untersuchungen inspizieren. Hierzu bedarf es lediglich eines minimalinvasiven Eingriffes, der für den Patienten keine weiteren Gefahren darstellt.

Zu Zwecken der Untersuchung im Magen-Darm-Bereich wird in der DE 101 27 850 A1 ein Endoskop beschrieben, das einen Hohlkanal umschließt, durch den eine MR-Spule hindurchführbar ist, die das distale Ende des Endoskops überragend positioniert werden kann, wobei die MR-Spule einen flexiblen elektrischen Leiter aufweist. Der elektrische Leiter ist von einem dillatierbaren Schlauch umgeben, der für die Formgebung der MR-Spule nach entsprechender intrakorporaler Positionierung verantwortlich ist. Zwar ermöglicht eine derartige Endoskopvorrichtung ein Erstellen hochqualitativer NMR-Aufnahmen von intrakorporalen Körperbereichen, doch wird eine derartige Untersuchung von dem Patienten als unangenehm empfunden.

Aus der WO 2005/024447 A1 geht eine Vorrichtung hervor, mit der ein intrakorporal einführbares Mittel, bspw. ein Katheter, mittels eines bildgebenden Magentresonanzverfahrens im Hinblick auf seine intrakorporale Position erfasst und überwacht werden kann. Hierzu wird an dem einführbaren Mittel ein Element mit einem Schwingkreis, bestehend aus einer HF-Spule sowie einer einstellbaren Kapazität, vorgesehen. Die Kapazität besteht aus einem halbleitenden lichtempfindlichen Material, wobei der Wert der Kapazität abhängig von einer Lichteinwirkung auf das Material einstellbar ist. Die Vorrichtung sieht weiterhin einen Lichtleiter vor, mit dem die Kapazität variabel belichtbar und damit die Resonanzfrequenz des Schwingkreises einstellbar ist. Während des Betriebes der Vorrichtung wird die Belichtung der Kapazität derart gesteuert, dass die Resonanzfrequenz des Schwingkreises mit der Anregungsfrequenz eines Magnetresonanzscanners übereinstimmt. Der derart abgestimmte Schwingkreis reagiert auf resonante Anregungen mit magnetischen Antwortsignalen, welche von dem Magnetresonanzscanner erfassbar sind und durch entsprechende Auswertung eine Überwachung der Position des mit dem einführbaren Mittel verbundenen Elementes erlauben.

In dem Artikel von Harald H. Quick et al. "Vascular Stents as RF Antennas for Intravascular MR Guidance and Imaging", in Magnetic Resonance in Medicine, 42, 1999, Seiten 738 -745, wird eine Studie vorgestellt, bei der eine auf einem bildgebenden Magnetresonanzverfahren (MR) und einem Radiofrequenzen empfangenden Stent beruhende aktive Visualisierung der vaskulären Position des Stents untersucht wird. Dabei dient der vaskulär implantierbare Stent als RF-Empfänger. In dem Artikel werden die Ergebnisse von folgenden drei unterschiedlichen Stent-Konfigurationen verglichen: 1. Rahmeantenne, 2. Diploantenne und 3. Hybridkonfiguration als Koaxialantenne. Die beschriebenen Experimente erlauben desweiteren Aussagen über die mit diesen Stent-Konfigurationen möglichen Ergebnisse bildgebender Magnetresonanzverfahren hinsichtlich a) der Erfassung der Verschiebung eines gefalteten Stents in Echteit, b) der Erfassung der Stententfaltung in Echtzeit, c) der Erzeugbarkeit von hochauflösenden intravaskulären Bildern des Läsionsbereichs und d) der Erfassung der Endothealialisation innerhalb des Stents.

In dem Artikel von Harald H. Quick et al. "Endourethral MRI", Magentic Resonance in Medicine, 45, 2001, Seiten 138 - 146, wird eine Studie beschrieben, die das Ziel verfolgt, ultra-hochauflösende Magnetresonanzaufnahmen von der weiblichen Harnröhre sowie dem umgebenden Gewebe zu ermöglichen. Hierfür werden zwei verschiedene HF-Spulenanordnungen beschrieben, die derart ausgeführt sind, dass sie in die Harnröhre einführbar sind.

Dem Artikel von Hui H. Qiu et al. "Measuring the Progression of Foreign-Body Reaction to Silicone Implants Using in Vivo MR Microscopy", in IEEE Transactions on Biomedical Engineering, Vol. 45, No. 7, July 1998, kann entnommen werden, dass HF-Spulennordnungen zur Erfassung und Untersuchung von Gewebewachstum mittels bildgegeber Magnetresonanzverfahren auch intrakorporal implantiert werden.

Schließlich gehen aus dem Artikel von lan Young,"MEMS MR Detector Coils", The IEE Measurement, Sensors, Instrumentation and NDT Profesional Network, Michael Farady House, Six Hills Way, Stevenage, Herts SG1 2AY, UK, verschiedene HF-Spulenanordnungen auf Silikonsubstratbasis hervor, die als Miniaturdetektoren im Rahmen von bildgegebenden Magentresonanzverfahren einsetzbar sind.

### Darstellung der Erfindung

Der Erfindung liegt die Aufgabe zugrunde, eine Vorrichtung zur NMR-Untersuchung intrakorporaler Körperbereiche einer Person mit einer Spulenanordnung, die in unmittelbarer Nähe zu einem intrakorporalen Körperbereich bringbar ist, der von einem stationären, magnetischen Polarisationsfeld B₀ sowie von einem RF-Wechselfeld durchsetzt wird, derart auszubilden, dass die Anwendung der Vorrichtung für eine Person nicht als unangenehm empfunden wird. Die Vorrichtung soll einfach, zuverlässig einsetzbar und kostengünstig sein. Zugleich besteht jedoch die Forderung nach hochqualitativen intrakorporalen Bildaufnahmen. Die Vorrichtung soll sich insbesondere zur Untersuchung des Magen-Darm-Traktes eignen.

Die Lösung der der Erfindung zugrunde liegenden Aufgabe ist in den nebengeordneten Ansprüchen 1 und 8 angegeben. Den Erfindungsgedanken vorteilhaft weiterbildende Merkmale sind Gegenstand der Unteransprüche sowie der Beschreibung unter Bezugnahme auf das Ausführungsbeispiel zu entnehmen.

Lösungsgemäß ist eine Vorrichtung zur NMR-Untersuchung gemäß den Merkmalen des Oberbegriffes des Anspruches 1 derart ausgebildet, dass die Spulenanordnung zum Empfang von MR-Echosignalen in einer gekapselten, autarken Einheit integriert ist, die eine von der Person verschluckbare Form und Größe aufweist. Vorzugsweise soll Form und Größe in Anlehnung an auf dem Markt erhältlichen oral einnehmbaren Pillen gerichtet sein. Innerhalb der gekapselten, autarken Einheit ist zudem eine Sendeeinheit integriert, die mit der Spulenanordnung verbunden ist und von Seiten der Spulenanordnung herrührende freie Induktionssignale an eine extrakorporal vorgesehene Empfängereinheit drahtlos übermittelt.

Die lösungsgemäße Vorrichtung gestattet eine ortsaufgelöste Bildaufnahme intrakorporaler Körperbereiche mittels NMR-Untersuchungstechnik ohne Zuhilfenahme Endoskop-unterstützter MR-Spulenpositionierungen innerhalb des Körpers, und dies mit gleicher und sogar besserer Bildauflösung und Bildqualität, d.h. erhöhtem Kontrastverhältnis, verglichen zu jenen NMR-Aufnahmen, die mit den auf dem Markt befindlichen MRI-Scanner-Systemen aufgenommen werden können.

Die der Erfindung zugrunde liegende Idee geht von einem HF-Spulensystem aus, das vollständig in eine biokompatible Matrix eingegossen ist, in der sämtliche Komponenten integriert sind, die für den Empfang von MR-Echosignalen aus dem jeweiligen Körperbereich notwendig sind, sowie für eine drahtlose Übermittlung der Echosignal-Informationen an eine extrakorporal vorgesehene Empfängereinheit erforderlich sind, in der die MR-Signalinformationen verstärkt und im weiteren in an sich bekannter Weise zur bildlichen Darstellung gebracht werden. Diese Komponenten umfassen in erster Linie eine in der biokompatiblen Matrix der autarken gekapselten Einheit integrierte Empfangsspule und eine Sendeeinheit zur drahtlosen Weiterleitung der MR-Echosignale an die extrakorporal vorgesehene Empfängereinheit. Die autarke, gekapselte Einheit weist vorzugsweise eine runde, glatte Oberfläche auf, durch die keinerlei Verletzungsgefahr für den Patienten bei oraler Einnahme darstellt. Der zu untersuchende Patient befindet sich nach Einnahme der gekapselten Einheit innerhalb eines Standard-Kernspin-Tomographen, durch den die für die NMR-Untersuchung erforderlichen Magnetfelder sowie das elektromagnetische Wechselfeld erzeugt werden. Die gekapselte Einheit bewegt sich auf natürlichem Wege nach oraler Einnahme durch die Speiseröhre in Richtung Magen, in dem die Einheit dem natürlichen Verdauungsprozess ausgesetzt ist. Bei der Wahl des biokompatiblen Materials zur Kapselung der vorstehenden elektrischen Einheiten ist darauf zu achten, dass das Material durch die chemisch aggressive Magensäure nicht angegriffen wird.

In einer bevorzugten Ausführungsform verfügt die gekapselte Einheit über wenigstens eine Magnetfeld-sensible Positioniereinheit, die durch Wechselwirkung mit einem extern vorherrschenden, elektrostatischen oder elektrodynamischen Feld räumlich ausrichtende Drehmomente auf die gekapselte autarke Einheit ausübt. Mit Hilfe der wenigstens einen Positioniereinheit ist es möglich, insbesondere während der Aufenthaltszeit im Magenbereich, mit Hilfe der ohnehin durch den Kernspin-Tomographen erzeugten Magnetfelder oder durch entsprechend zusätzlich angelegte Steuermagnetfelder, die räumliche Lage der gekapselten Einheit zu manipulieren, um einerseits die autarke gekapselte Einheit zu navigieren und andererseits die in der Einheit integrierte MR-Empfangsspule jeweils für einen optimierten Empfang der MR-Echosignale aus dem zu untersuchenden Körperbereich auszurichten.

Nach Aufenthalt der gekapselten autarken Einheit innerhalb des Magenbereiches gelangt die Einheit im Rahmen der körpereigenen natürlichen Peristaltik durch die unterschiedlichen Darmabschnitte, bis sie letztlich über den natürlichen Ausgang ausgeschieden wird. Da die äußere Beschaffenheit der Einheit durch die aus biokompatiblem Material bestehende Kapselung vollständig fluiddicht ausgebildet ist, besteht die Möglichkeit der Autoklavierung, d.h. vollständigen Sterilisation, so dass die gekapselte, autarke Einheit durchaus einer Wiederverwendung zugeführt werden kann. Gleichwohl ist es aufgrund der kostengünstigen Herstellung ebenfalls möglich, die lösungsgemäße Vorrichtung als Einwegartikel anzubieten.

### Kurze Beschreibung der Erfindung

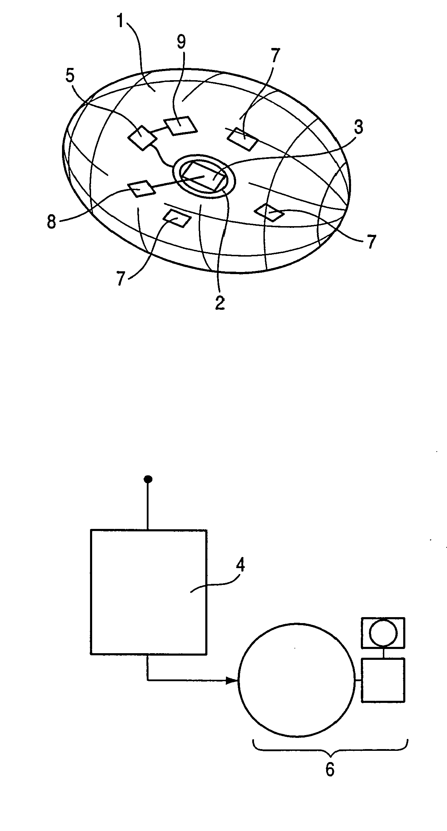

Die Erfindung wird nachstehend ohne Beschränkung des allgemeinen Erfindungsgedankens anhand eines Ausführungsbeispiels unter Bezugnahme auf die einzige Zeichnung exemplarisch beschrieben.

### Wege zur Ausführung der Erfindung, gewerbliche Verwendbarkeit

In der einzigen Figur ist stark schematisiert und aus Gründen einer besseren Übersichtlichkeit eine pillenförmig ausgebildete gekapselte, autarke Einheit 1 dargestellt, die über eine glatte Oberfläche verfügt und im wesentlichen aus biokompatiblem Material gefertigt ist. Im Inneren der Einheit 1 befindet sich wenigstens eine RF-Empfangsspule 2, mit der es möglich ist, durch RF-Feldenergie-Absorption hervorgerufene Kernspin-Prozess-korrelierte Echosignale aus einem im Rahmen einer NMR-Untersuchung untersuchten intrakorporalen Körperbereich zu empfangen. Für eine möglichst breitbandige Abstimmung der Empfangsspule 2 auf die seitens des Kernspin-Tomographen vorgegebene Frequenz des elektromagnetischen Wechselfeldes ist eine ebenfalls in der biokompatiblen Matrix der Einheit 1 eingegossene, miniaturisiert ausgeführten Beschaltung 3 vorgesehen, bestehend aus Kondensatoren und Widerständen. Um die von der Empfangsspule 2 empfangenen MR-Informationen an eine extern bzw. extrakorporal vorgesehene Empfängereinheit 4 drahtlos weiterzuleiten, ist innerhalb der gekapselten, autarken Einheit 1 ein miniaturisierter Sender 5 vorgesehen, der das von der Spule 2 herrührende freie Induktionssignal an die Empfängereinheit 4 übermittelt, in der das Signal verstärkt und vorzugsweise an ein Empfangs-/Sendekabelsystem des Kernspin-Tomographen 6 eingekoppelt wird.

Ferner ist innerhalb der biokompatiblen Matrix der Einheit 1 wenigstens eine, vorzugsweise drei Magnetfeld-sensible Positioniereinheiten 7 vorgesehen, mit denen es möglich ist, die gekapselte, autarke Einheit 1 durch ein extern angelegtes Steuermagnetfeld räumlich zu positionieren, um auf diese Weise die Einheit 1 an bestimmte bzw. bevorzugte Körperbereiche navigieren zu können, die es zu untersuchen gilt. Die Positioniereinheiten 7 sind vorzugsweise als miniaturisierte Permanentmagnete, Induktivitäten oder als magnetisierbare Einheiten ausgebildet, die durch Wechselwirkung mit einem extern vorherrschenden elektrostatischen oder elektrodynamischen Feld auf die gekapselte autarke Einheit räumlich ausrichtende Drehmomente zu erzeugen vermögen.

Um zu verhindern, dass durch induktive Energieabsorption innerhalb der Empfängerspule 2 eine Überhitzung stattfindet, ist zusätzlich ein Überhitzungsschutz 8 in der Einheit 1 vorgesehen, beispielsweise in Form einer thermo-sensiblen Kontaktstelle, durch die der Stromkreis der in der Einheit 1 integrierten elektrischen Beschaltung 3 bei Überhitzung unterbrochen werden kann. Zur Energieversorgung ist zudem ein kleiner Akku 9 in der Matrix der Einheit 1 integriert, um die für die Aussendung der MR-Echosignalinformationen über die Sendeeinheit 5 sicher zu stellen.

### Bezugszeichenliste

- 1: Gekapselte, autarke Einheit
- 2: MR-Spulenanordnung
- 3: Beschaltung
- 4: Externe Empfangseinheit
- 5: Sendeeinheit
- 6: Kernspin-Tomograph mit Bildauswerteeinheit und Bilddarstellungseinheit
- 7: Positioniereinheit
- 8: Thermoschutzeinheit
- 9: Energiequelle, Akku

## Patentansprüche

1. Vorrichtung zur NMR-Untersuchung intrakorporaler Körperbereiche einer Person mit einer Spulenanordnung, die in unmittelbare Nähe zu einem intrakorporalen Körperbereich bringbar ist, der von einem stationären, magnetischen Polarisationsfeld B₀ sowie von einem RF-Wechselfeld durchsetzt wird,
**dadurch gekennzeichnet, dass** die Spulenanordnung in einer gekapselten, autarken Einheit integriert ist, die eine von der Person verschluckbare Form und Größe aufweist,
dass in der gekapselten, autarken Einheit eine Sendeeinheit integriert ist, die mit der Spulenanordnung verbunden ist und von Seiten der Spulenanordnung herrührende Induktionssignale an eine extrakorporal vorgesehene Empfängereinheit drahtlos übermittelt.

2. Vorrichtung nach Anspruch 1,
**dadurch gekennzeichnet, dass** das stationäre, magnetische Polarisationsfeld B₀ sowie das RF-Wechselfeld mittels einer Standard-Tomographieanordnung (MRI-Scanner) erzeugbar sind.

3. Vorrichtung nach Anspruch 1 oder 2,
**dadurch gekennzeichnet, dass** die gekapselte, autarke Einheit aus biokompatiblen Material besteht.

4. Vorrichtung nach einem der Ansprüche1 bis 3,
**dadurch gekennzeichnet, dass** in der gekapselten autarken Einheit wenigstens eine magnetfeldsensible Positioniereinheit integriert ist, die durch Wechselwirkung mit einem extern vorherrschenden, elektrostatischen oder elektrodynamischen Feld räumlich ausrichtende Drehmomente auf die gekapselte autarke Einheit ausübt.

5. Vorrichtung nach Anspruch 4,
**dadurch gekennzeichnet, dass** die magnetfeldsensible Positioniereinheit eine Induktivität, ein Permanentmagnet oder eine magnetisierbare Einheit ist.

6. Vorrichtung nach Anspruch 5,
**dadurch gekennzeichnet, dass** an wenigstens drei räumlich getrennten Stellen innerhalb der gekapselten, autarken Einheit eine magnetfeldsensible Positioniereinheit vorgesehen ist.

7. Vorrichtung nach einem der Ansprüche 1 bis 6,
**dadurch gekennzeichnet, dass** die Spulenanordnung mit einem Überhitzungsschutz versehen ist.

8. Verwendung der Vorrichtung nach einem der Ansprüche 1 bis 7 zur NMR-Untersuchung des Magen-Darm-Traktes einer Person, durch Verschlucken der gekapselten, autarken Einheit, die im Wege der natürlichen Peristaltik nach oraler Einnahme intrakorporale NMR-Bildaufnahmen ermöglicht.
